# FASCICULE DE BREVET EUROPEEN

(11) **EP 0 980 525 B1**
(45) Date de publication et mention de la délivrance du brevet: **20.08.2003**
(21) Numéro de dépôt: 98922887.9
(22) Date de dépôt: 28.04.1998
(51) Int. Cl.: G01N 33/564, G01N 33/92, G01N 33/68

(54) **METHODES POUR LA DETECTION DE PATHOLOGIES AUTO-IMMUNES**
VERFAHREN ZUM NACHWEIS VON AUTOIMMUN-PATHOLOGIEN
METHODS FOR DETECTING AUTOIMMUNE PATHOLOGIES

(30) Priorité: 28.04.1997 FR 9705228
(43) Date de publication de la demande: 23.02.2000
(73) Titulaire: INSTITUT NATIONAL DE LA SANTE ET DE LA RECHERCHE MEDICALE (INSERM), 75654 Paris Cédex 13 (FR)
(72) Inventeur: PETRY, Klaus, F-33760 Targon (FR); BOULLERNE, Anne, F-17100 Saintes (FR)
(74) Mandataire: Peaucelle, Chantal
(86) Numéro de dépôt international: FR9800853
(87) Numéro de publication internationale: WO98049558

(56) Documents cités:
- A.I. BOULLERNE ET AL.: "Indirect evidence for nitric oxide involvement in multiple sclerosos by characterization of circulating antibodies directed against conjugated S-nitrosocysteine" JOURNAL OF NEUROIMMMUNOLOGY, vol. 60, 1995, AMSTERDAM NL, pages 117-124, XP002052448 cité dans la demande
- A.I. BOULLERNE ET AL.: "Circulating antibodies directed against conjugated fatty acids in sera of patients with multiple sclerosis." JOURNAL OF NEUROIMMMUNOLOGY, vol. 65, 1996, AMSTERDAM NL, pages 75-81, XP002052449 cité dans la demande

## Description

L'invention a pour objet des moyens pour la détection précoce de pathologies auto-immunes inflammatoires du système nerveux central ou périphérique.

Elle concerne en particulier des outils et méthodes, permettant de prédire de manière précoce la survenue de poussées cliniques, ou de lésions, telles qu'observées dans les maladies auto-immunes du système nerveux central ou périphérique, par exemple dans le cas de la sclérose en plaques, ou des neuropathies périphériques associées à des gammopathies à IgM, ou de la maladie de Guillem-Barré.

On rappelle que la sclérose en plaques (en abrégé SEP), affection démyélinisante du système nerveux central, est caractérisée par la survenue des lésions inflammatoires et démyélinisantes disséminées ("plaques") dans la substance blanche encéphalique et médullaire.

Les mécanismes de la constitution des nouvelles lésions de la SEP commencent à être bien connus. Ils comprennent les étapes de : 1) présentation d'un antigène par des cellules de la ligne macrophagique aux lymphocytes T auxiliaires en présence de cytokines pro-inflammatoires, 2) prolifération des lymphocytes et leur différention, 3) rupture de la barrière hémato-encéphalique et passage de ces cellules immunocompétentes dans le parenchyme qui provoquent une démyélinisation.

Les lésions peuvent évoluer vers une perte axonale et/ou une gliose astrocytaire.

On distingue, trois formes évolutives principales de SEP, qui se caractérisent par leur évolution clinique, mais aussi par la localisation des lésions, les anomalies d'imagerie en résonance magnétique (IRM) et les perturbations immunologiques associées.

La forme la plus fréquente est la forme rémittente caractérisée par une évolution par poussées espacées de rémissions plus ou moins complètes.

La forme progressive primaire est caractérisée par une évolution régulière et progressive depuis le début de la maladie.

Dans la forme progressive secondaire, une phase d'évolution progressive succède à une phase rémittente.

La fréquence des poussées est très variable d'un patient à l'autre et leur sévérité n'est pas prévisible.

Ainsi, la rareté des poussées est habituellement un facteur prédictif d'un faible handicap. Certains patients développeront cependant de lourdes séquelles au cours d'un petit nombre de poussées. Au contraire, d'autres patients ne garderont que peu de séquelles malgré une fréquence élevée de poussées. Les études IRM ont montré que la plupart des patients présentent une activité de leur maladie (apparition de lésions nouvelles) en dehors des poussées cliniques, mais que cette activité est très variable d'un patient à l'autre.

Ce processus de rémission, suivi de poussées se retrouve dans les autres pathologies auto-immunes inflammatoires évoquées ci-dessus, par exemple dans la polyarthrite rhumatoïde.

Les thérapeutiques actuelles consistent essentiellement en un traitement des symptômes cliniques. Elles consistent en effet à la suppression des poussées par des immunosuppresseurs ou par l'administration de corticoïdes.

Ces thérapeutiques méritent cependant d'être prescrites à bon escient étant donné les effets secondaires non négligeables des médicaments administrés et leur coût élevé.

Cette situation a conduit à rechercher des marqueurs biologiques permettant de prédire la survenue d'une poussée.

Pour la SEP par exemple, plusieurs stratégies ont été appliquées.

Ainsi, il a été montré que les patients atteints de formes bénignes de SEP avaient tendance à avoir une accumulation plus faible de nouvelles lésions en IRM sur une année, mais ceci est une constatation rétrospective peu utile en pratique thérapeutique.

La production de TNF-α par les cellules mononucléaires circulantes du sang a été considérée comme un marqueur prédictif de la survenue d'une poussée clinique ou d'une nouvelle lésion, mais les données d'une étude à l'autre restent contradictoires et ce marqueur ne permet de prévoir ni la sévérité, ni les séquelles de la poussée. Ce marqueur n'est donc pas utilisé en routine.

Les travaux des inventeurs dans le domaine de ces neuropathies (1) et (2) les avaient amenés à mettre en évidence des taux élevés d'anticorps anti-NO-cystéine ou encore d'anticorps anti-acides gras chez les malades atteints d'affections de ce type.

En utilisant un modèle animal d'encéphalite auto-immune expérimentale (EAE), dont les symptômes sont proches de la SEP humaine, les inventeurs ont mis en évidence le rôle de ces anticorps comme marqueurs précoces de l'évolution de la maladie.

Les expériences réalisées ont permis de généraliser ces résultats aux affections auto-immunes inflammatoires du système nerveux central ou périphérique.

L'invention a donc pour but de fournir de nouveaux marqueurs et tests permettant de prédire la survenue, la sévérité et l'évolution d'une poussée et de procéder ainsi à un traitement préventif précis des poussées.

L'invention vise ainsi l'utilisation *in vitro* d'anticorps tels que développés durant une pathologie auto-immune inflammatoire du système nerveux central ou périphérique, ces anticorps appartenant au groupe constitué par les anticorps anti-NO-P, où P représente une protéine, une protéine sous forme recombinante, un peptide, ou un acide aminé, capable d'accepter NO, ou les anticorps anti-acides gras, comme marqueurs prédictifs d'une poussée clinique ou d'une nouvelle lésion de ladite pathologie.

Dans les anticorps anti-NO-P, utilisés selon l'invention comme marqueurs, P représente notamment, en tant que protéine, une glycoprotéine associée à la myéline (MAG) ou une glycoprotéine majeure d'oligodendrocytes (MOG) ou, comme acide aminé, P représente un résidu cystéine.

D'une manière générale, la protéine représentée par P peut être, sous forme recombinante.

Dans les anticorps anti-acide gras, celui-ci est choisi par exemple parmi l'acide laurique, l'acide palmitique, l'acide palmitoléique, l'acide stéarique, l'acide oléique, l'acide linoléique, l'acide linoléique, l'acide arachidonique, l'acide eicosapentanoïque et l'acide docosahexaénoïque.

D'une manière inattendue, la présence de ces anticorps dans le sérum des malades peut être corrélée à la survenue ultérieure d'une poussée ou d'une lésion.

Les expériences réalisées sur un modèle EAE, qui sont rapportées dans les exemples donnés ci-après, permettent d'établir une corrélation entre le taux d'anticorps anti-NO-cystéine, le déficit fonctionnel mesuré par la force musculaire et le degré de démyélinisation du cervelet.

De même, une corrélation a été établie entre la production d'anticorps anti-acides gras à un stade préclinique et l'évolution ultérieure de la maladie.

L'invention fournit également un test de diagnostic précoce de la survenue de poussées et de lésions dans une pathologie auto-immune inflammatoire du système nerveux central ou périphérique, caractérisé en ce que les marqueurs du processus inflammatoire, de démyélinisation, et de déficit musculaire sont constitués par des anticorps anti-NO-P, où P est tel que défini ci-dessus, et/ou anti-acide gras.

Ce test comprend avantageusement la mesure qualitative et quantitative des anticorps anti-NO-P et/ou anti-acide gras dans le sérum d'un patient par mise en contact du sérum avec un antigène nitrosylé comportant P ou un acide gras, et la révélation de la réaction antigène-anticorps lorsqu'elle s'est produite.

La mise en contact comprend l'incubation de l'échantillon testé et de l'antigène à une température appropriée pour la formation du complexe antigène-anticorps. On opère généralement à une température de l'ordre de 37°C.

L'antigène est habituellement couplé à une protéine porteuse.

Des protéines communément utilisées comprennent la KLH (hémocyanine de patelle) ou la BSA (albumine de sérum bovin), utilisée sous forme délipidée lorsque le test est appliqué à la détermination d'anticorps anti-acide gras.

La protéine porteuse est séparée généralement de l'haptène par un bras d'écartement. Il s'agit par exemple de glutaraldéhyde.

L'antigène comprend de préférence, comme entité P, un résidu cystéine et, comme acide gras, un acide gras quelconque, choisi par exemple parmi l'acide laurique, l'acide palmitique, l'acide palmitoléique, l'acide stéarique, l'acide oléique, l'acide linoléique, l'acide linoléique, l'acide arachidonique, l'acide eicosapentanoïque et l'acide docosahexaénoïque.

L'antigène est préférentiellement fixé sur un support tel qu'une microplaque à multipuits.

Dans un mode préféré de réalisation de l'invention, le test correspond à une méthode ELISA, un deuxième anticorps étant fixé à l'antigène et révélé selon les techniques connues.

Parallèlement aux études sérologiques, l'invention permet d'une part une caractérisation antigénique des molécules myéliniques nitrosylées, d'autre part une analyse moléculaire par exemple de cytokines, métallo-protéinases, radicaux libres, des interactions des cellules microgliales et des cellules du SNC (astrocytes) impliquées dans les lésions myéliniques.

La caractérisation antigénique est effectuée avantageusement sur des protéines recombinantes nitrosylées. Différentes constructions permettent ainsi, par exemple, la synthèse de fragments de la MOG recombinante. Afin de distinguer les différentes formes et stades évolutifs de SEP, on a alors avantageusement recours à banque de sérums de patients contre ces protéines recombinantes nitrosylées.

D'autres caractéristiques et avantages de l'invention sont donnés dans les exemples qui suivent.

Dans ces exemples, il est fait référence aux figures 1 à 4, qui représentent respectivement,
- la figure 1, la densité optique (taux d'anticorps anti-NO-cystéine), et le déficit fonctionnel mesurés durant une EAE chez le rat,
- la figure 2, des photographies au microscope optique de sections du cervelet durant l'EAE,
- la figure 3, les coefficients de corrélation entre les anticorps anti-NO-cystéine au 6ème jour après l'induction de l'EAE et la force musculaire, et
- figure 4, la photo d'un Western blot révélant des antigènes myéliniques humains nitrosylés par les sérums de patients atteints de SEP.

### Exemple 1 : Matériels et méthodes

### a) Couplage de la cystéine à la BSA (bovine serum albumin ou albumine de sérum bovin) via le glutaraldéhyde.

Pour un rapport de couplage de 9, on procède comme suit :

On prépare tout d'abord
- 5 ml d'une solution de glutaraldéhyde à 5 % (1 ml de glutaraldéhyde à 2,5 % + 4 ml d'eau) ;
- du tampon d'acétate de sodium (1,5 M, pH 8-8,5)
- une solution de borohydrure de Na (eau dans un tube de 3 ml avec une petite spatulée de NaBH₄)

On pèse séparément 3 mg de L-cystéine et 10 mg de BSA.

On reprend la L-cystéine dans 1 ml de tampon acétate, puis on ajoute 1 µl de tyrosine tritiée (³H) 1,9 TBq/mmole dans la solution de L-cystéine ; et extemporanément 100 µl de glutaraldéhyde 5 % ainsi que la BSA. Le mélange est soumis à un traitement au Vortex. On laisse jaunir le mélange pendant au moins 5 min, tout en poursuivant de temps en temps le traitement au vortex.

On ajoute ensuite 100 µl de borohydrure de sodium et on laisse la réaction s'effectuer ensuite pendant 30 min environ en agitant régulièrement.

On soumet ensuite le mélange réactionnel à une dialyse pendant 24 h contre de l'eau, en utilisant 3 bains.

La radioactivité est comptée sur des aliquots de 100 µl pris avant et après la dialyse pour calculer le taux de couplage de l'acide aminé à la BSA. La concentration de BSA est évaluée en mesurant la densité optique (DO) à 280 nm. Le rapport de couplage est de 9. Ce rapport est conservé lorsqu'on multiplie par 2 tous les volumes.

Le produit de couplage formé, désigné ci-après en abrégé par Cys-g-BSA, où g = glutaraldéhyde, répond à la formule ;

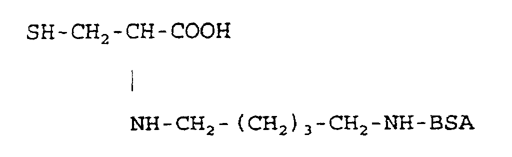

### b) Nitrosylation de Cys-g BSA avec NaNO₂

On pèse 2 mg de NaNO₂ pour 1 mg de Cys-g-BSA dans un tube à l'abri de la lumière.

On ajoute ensuite NaNO₂ à 1 ml de Cys-g-BSA, puis HCl (41,7 µl de HCl 12N concentré pour 1 ml). On observe un changement de coloration de la solution vers le jaune pâle. Le mélange est soumis à incubation 4 h à 37°C sous agitation, puis est dialysé pendant 24 h contre le tampon Tris 0,01M, pH 7,4 (HCl). Pour les 2 bains suivants, on utilise de l'eau.

Le conjugué NO-Cys-g-BSA est analysé par spectrophotométrie entre 240 et 500 nm : le NO absorbe entre 320 et 360 nm (3).

### c) Synthèse de BSA-g

On ajoute 100 mg de BSA dans 1 ml de tampon acétate 3M, pH 8, et 2 ml d'eau.

On ajoute ensuite 1 ml de glutaraldéhyde à 1 % et, après 5 min, tout en soumettant de temps en temps le mélange à un traitement au vortex, on ajoute 200 µl de NaBH₄.

On laisse la réaction s'effectuer pendant 30 min en agitant régulièrement, et on dialyse le mélange réactionnel pendant 24 h contre H₂O en utilisant 3 bains.

### d) Préparation de BSA délipidée (dBSA)

On pèse 8 x 1 g de BSA dans des tubes de 50 ml, puis on ajoute 10 ml d'H₂O et on soumet à un traitement au Vortex pour une dissolution complète. On ajoute ensuite 10 ml de fréon (1,1,2-trichlorotrifluoroéthane C₂Cl₃F₃) conservé à 4° C.

Après un vortex de 1 min pour mélanger les 2 phases (le tube est rempli de mousse blanche), on centrifuge 10 min à 2000 rpm pour séparer les 2 phases et on récupère la phase supérieure aqueuse, qu'on met à lyophiliser dans un grand bécher en couvrant de parafilm ou dans 8 tubes de 50 ml congelés penchés.

### e) délipidation du sérum ou d'une protéine

La délipidation se fait volume à volume avec du fréon, en agitant 2-3 minutes, puis en centrifugeant 15 min à 10000 rpm. La phase aqueuse (couche supérieure) est récupérée.

### f) Synthèse des acides gras-dBSA par l'éthylchloroformate

Dans des tubes préalablement délipidés au méthanol, on pèse ou on introduit avec une pipette 1 mg ; 2,5 mg- ou 5 mg d'acides gras, selon le rapport de couplage désiré, respectivement 10 ; 20 et 40.

On pèse ensuite 20 mg de dBSA

On reprend l'acide gras dans 1 ml de méthanol anhydre + 10 µl de triéthylamine anhydre + 1 µl de palmitate C¹⁴ et on laisse ½ h minimum sur silicagel. On reprend ensuite la dBSA dans 2 ml de phosphate CaCl₂ 1 mM + 10 µl de triéthylamine.

En opérant en chambre froide, on ajoute 200 µl d'éthylchloroformate dilué au 1/16 dans du diméthylformamide anhydre (12,5 µl d'éthylchloroformate dans 187,5 µl de diméthylformamide) dans l'acide gras. On soumet le mélange réactionnel à un traitement au vortex 30 sec et on laisse 3 min (activation des COOH). On ajoute ensuite la dBSA en agitant avec le vortex.

On ajoute 2 ml de la solution du 1er bain de dialyse pour favoriser la solubilité du conjugué, puis on mesure la radioactivité (10 µl d'échantillon + 90 µl d'H₂O dans 2 ml de liquide scintillant). On procède ensuite à une dialyse 12 heures dans un mélange 1/3 méthanol, 1/3 diméthylformamide et 1/3 phosphate CaCl₂, 1 mM, puis on effectue 2 bains de phosphate CaCl₂, 1mM.

On mesure la radioactivité avec 100 µl d'échantillon dans 2 ml de liquide scintillant.

On établit un spectre de 240 à 320 nm afin de déterminer sa concentration en relevant les DO à 300 et 280 nm (ε BSA : 34500). Les conjugués acides gras se conservent plusieurs mois à -80°C.

### g) Test ELISA avec NO-Cys-g-BSA

On utilise des plaques Nunc Maxisorp® à 96 puits (test à 200 µl). Le revêtement des plaques est effectué à l'aide de 10 µg de NO-Cys-g-BSA/ml dans du tampon carbonate pH 9,6 (témoin BSA-g) pendant environ 16h, à 4°C, sous agitation.

Pour saturer tous les sites non spécifiques, on utilise comme tampon PBS Tween 20®, auquel on ajoute 10% de glycérol et 1 mg/ml de BSA (tampon X) pendant 1 h à 37°C.

On procède à 2 rinçages au PBS Tween 20®.

Le sérum ou les anticorps du patient, ainsi que les témoins, sont dilués à 1/500 dans le tampon X additionné de 0,1 % BSA-g.

On procède à 2 rinçages au PBS Tween®.

On utilise, comme anticorps secondaire, un anticorps anti-IgM humain (anti-IgM, IgA, IgG de Pasteur Diagnostics), dilué à 1/5000 dans le tampon PBS Tween® additionné de 1 mg/ml de BSA, pendant 1 h à 37°C. On procède à 3 rinçages au PBS Tween®.

La révélation est effectuée à l'aide de OPD 2g/l en tampon citrate phosphate + H₂O₂ 0,1 % pendant 10 min à l'obscurité.

Pour arrêter la réaction, on ajoute 50 µl de H₂SO₄ 4N. Les lectures de la DO sont effectuées à 490 nm.

### h) Test ELISA avec Ole-dBSA

### (Ole = acide oléique)

### - Tests pour les malades

On utilise des plaques de polystyrène Polysorp®, Nunc qu'on recouvre avec 200 µl d'une solution contenant le conjugué acide gras -dBSA ou la dBSA, à une concentration de 50 µg/ml dans du tampon carbonate (pH 9,6) + 1 mM de CaCl₂, pendant 16 h à 4°C, sous agitation (témoin : dBSA).

On n'effectue pas de saturation.

Les plaques sont rincées 2 fois avec 1 mM CaCl₂ et du PBS.

On ajoute l'anticorps I (AcI) dilué au Y₂₅₀ dans du tampon PBS (450 mM, NaCl), CaCl₂ 1 mM + 1 mg/ml de BSA pendant 2 h à 37°C. On effectue 2 rinçages avec PBS + CaCl₂ 1 mM.

On ajoute le 2ème anticorps (AcII) dilué au 1/5000 (anti-IgM, anti-IgA et anti-IgG humain, Institut Pasteur, Diagnostics) dans du tampon PBS + CaCl₂ 1 mM + 1 mg/ml de dBSA, pendant 1 h à 37°C.
On procède à 3 rinçages avec PBS-Tween ^{R} et à une révélation avec OPD 1 g/l + H₂O₂ 0,05 % pendant 10 min (demi-obscurité).

On arrête la réaction par addition de H₂SO₄ 4N et on procède à la lecture de la DO à 490 nm.

### - Tests pour les rats.

On opère comme indiqué ci-dessus mais Ac I est dilué au 1/500 et AcII (anti-IgM et anti-IgG de rat, Jackson Immunoresearch) est utilisé à raison de 0,12 mg/l de tampon.

### i) Tampons

### Acétate pH 8

| | |
|---|---|
| 3 M | 12,3 g/ 50 ml |

### HCl 0,5 M

41,7 µl/ml

### PBS pH 7,2

| | |
|---|---|
| NaCl | 9 g/l d'eau |
| Na₂HPO₄ | 1,42 g/l d'eau |
| NaH₂PO₄, H₂O | 0,276 g/l d'eau |

### PBS-Tween

50 µl de Tween 20®/l de PBS
Revêtement (tampon de carbonate pour test ELISA)

| | |
|---|---|
| Na₂CO₃ | 2,65 g/500 ml d'eau |
| NaHCO₃ | 4,2 g/ l d'eau |

Le pH est ajusté à 9,6 en versant Na₂CO₃ dans NaHCO₃

### Citrate phosphate pH 5

| | |
|---|---|
| citrate de Na | 10,5 g/l d'eau |
| Na₂HPO₄ | 17,8 g/l d'eau |

### Orthophénylène diamine

| | |
|---|---|
| OPD | 4 g/100 ml d'eau |

### Exemple 2: Etude de l'encéphalite auto-immune expérimentale (EAE) chez le rat

### . corrélation entre le taux d'anticorps anti-NO cystéine et l'intensité du déficit musculaire ultérieur et la démyélinisation ultérieure.

En opérant selon Wekerle (4) on provoque chez le rat Lewis, une encéphalite autoimmune expérimentale active par injection d'un peptide de protéine basique myélinique (acide aminé en position 68 à acide aminé en position 84).

Au cours d'une étude suivie sur 4 semaines, trois paramètres ont été analysés et corrélés : (a) le taux d'anticorps sérique anti-NO cystéine, (b) le déficit fonctionnel déterminé par la force musculaire durant le développement de l'EAE et (c) le degré de démyélinisation du cervelet.

Dans une première étape, on mesure par ELISA le taux d'anticorps sériques anti- NO-cystéine. Les valeurs sont exprimées en DO moyenne +/- S.E.M.

Les résultats obtenus sont rapportés sur la figure la qui donne la DO mesurée.

Dès le 6ème jour après l'induction, on observe une réponse IgM anti-NO-cystéine significative, dans les sérums des animaux EAE, par rapport aux témoins. La DO est en effet de 0,51 ± 0,05 (moyenne ± S.D; intervalle de 0,32 à 0,72) contre 0,13 ± 0,01 (intervalle de 0,08 à 0,15) chez les témoins [F_{4,48} = 21,43; p < 0,001].

Les essais de compétition montrent que les anticorps sont spécifiquement dirigés contre l'épitope NO-cystéine.

Dans les sérums des rats avec EAE, on n'observe aucune réponse IgG, mais seulement une réponse IgM dirigée contre la NO-cystéine, cette réponse n'étant plus détectable ensuite durant la EAE.

Dans une deuxième étape, on examine si l'altération des titres en anticorps anti-NO-cystéine est associée aux variations de signes cliniques, en déterminant les variations de la force musculaire.des membres postérieurs .

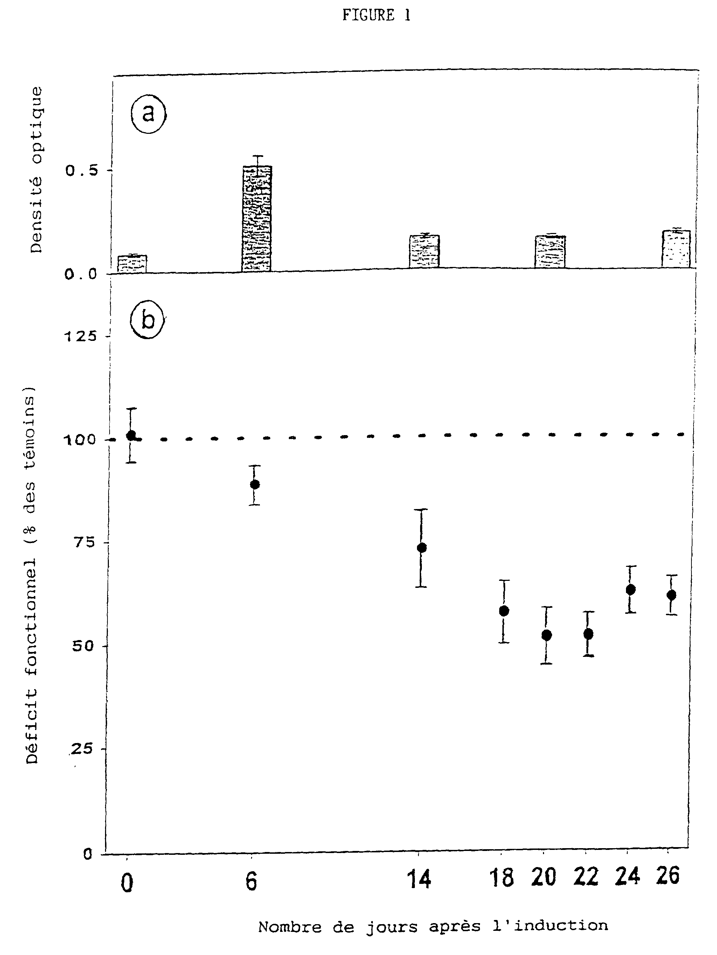

On a représenté sur la figure 1b les résultats obtenus, les déficits musculaires étant exprimés en % de la force musculaire des membres postérieurs des animaux témoins (n=5). Les valeurs données sont des moyennes ± S.E.M..

On a effectué 10 mesures sur chaque animal (n=9) par jour.

Le déficit fonctionnel est significatif au 18ième jour après l'injection et persiste jusqu'au 26ème jour.

Les rats EAE montrent une perte importante de leur force musculaire de l'ordre de 50 à 60 % par rapport aux animaux témoins.

On observe également chez les rats EAE, par rapport aux témoins, une diminution du poids corporel au moment où les signes cliniques apparaissent, mais aucune corrélation n'a été établie entre les signes cliniques et le poids corporel.

### . corrélation entre le taux d'anticorps et la démyélinisation du cervelet.

On mesure à la fin de l'étude l'étendue de la démyélinisation chez les animaux EAE.

On procède à une coloration spécifique de la myéline en opérant selon la méthode de SPIELMEYER (1).

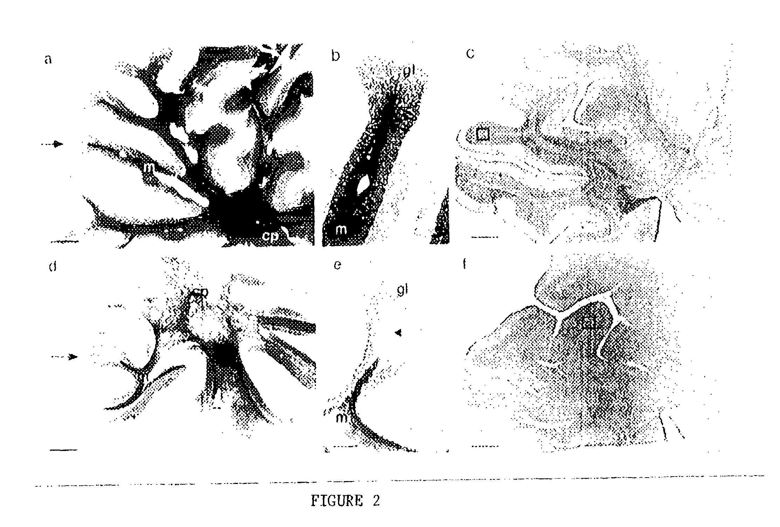

Sur la figure 2 on a représenté les photos au microscope de sections sagittales du cervelet de témoins (a, b, c) et de rats EAE (d, e, f).

Les flèches sur les figures a) et d) indiquent les parties représentées, respectivement, sur les figures b) et e) .

Les sections sagittales adjacentes sont immunocolorées à l'aide d'anticorps polyclonaux dirigés vers la protéine basique de myéline (PBM) (c,f).

La quantification de la myéline est effectuée en déterminant la réaction immunohistochimique avec les anticorps anti-PBM en utilisant le système Samba 2640 (Alcatel, TITN, Answare, France). Pour chaque animal, on étude 4 sections, en mesurant la DO moyenne (DOm) par surface définie ( ). Pour chaque section, le marquage de la PBM est mesuré dans la partie intérieure de la couche granulaire à l'intérieur de 3 lobes différents (4, 6, 8). Comme valeurs témoins, on utilise la couche moléculaire du cervelet connue pour contenir principalement des fibres dépourvues de myéline. Les données quantitatives sont exprimées en unités arbitraires (DOn/surface). Les symboles utilisés ont les significations suivantes : cp = pédoncule du cervelet ; m = moelle du cervelet ; gl = couche granulaire du cortex du cervelet ; la tête de la flèche indique la partie distale de la moelle du cervelet. Les barres représentent 350 µm pour (a, c, d, f) et 85 µm pour les détails donnés sur b et e.

La diminution de fibres de myéline apparaît la plus prononcée dans le pédoncule et la moelle du cervelet, ainsi que dans le premier tiers de la couche granulaire du cortex du cervelet.

De plus, l'importance de la maladie apparaît corrélée avec le processus de démyélinisation comme montré par la disparition complète des fibres de myéline dans la moelle distale en contact avec le cortex du cervelet des animaux les plus affectés (fig. 2e).

L'analyse quantitative du marquage immunohistochimique de la myéline du cervelet avec les anticorps anti-protéine basique de myéline confirme les résultats de la disparition de myéline dans le cervelet (fig. 2 f) .

Les animaux EAE les plus affectés présentent une diminution de 28 % de PBM par rapport aux rats témoins (df = 8 ; t = 2,64 ; O = 002).

### . coefficient de corrélation entre les anticorps anti-NO-cystéine au 6ème jour et la force musculaire

Les résultats obtenus montrent que le taux d'anticorps anti-NO-cystéine permet de prédire les signes pathophysiologiques dans l'EAE.

En réalité, un rapport inverse direct existe entre d'une part le taux d'anticorps anti-NO-cystéine au 6ème jour après l'induction et d'autre part la force musculaire des membres postérieurs au 22ème jour (Bravais Pearson coefficient, r=-0,72 ; p = 0,016) à 24 dpi (r=0,81 ; p = 0,008) à 26 dpi (r=0,96 ; p = 0,0001). et les valeurs de myéline des rats EAE (r = 0,77 ; p = 0,014).

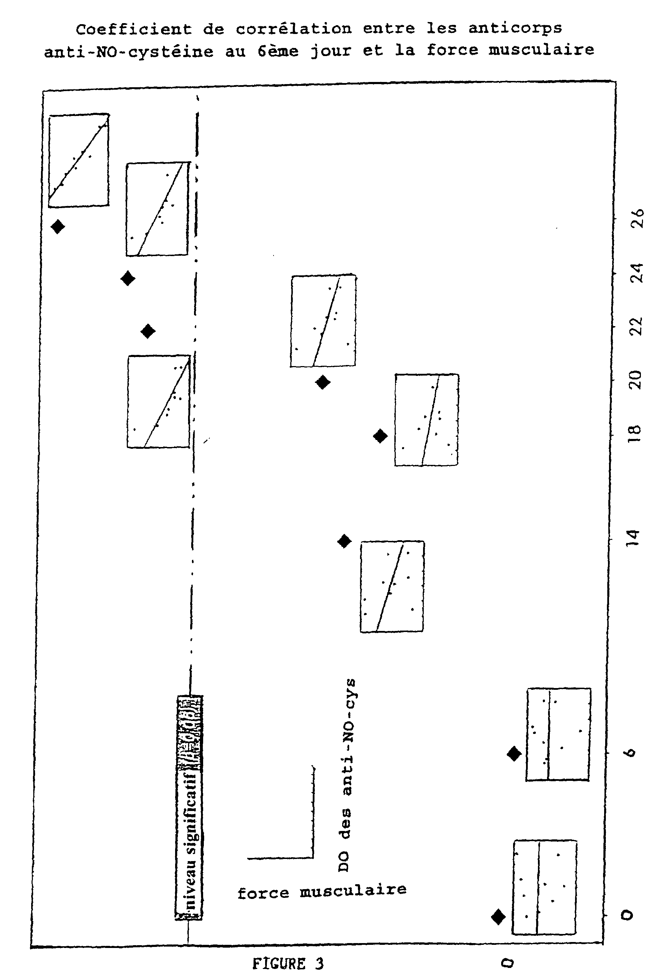

Sur la figure 3, on a représenté le coefficient de corrélation entre le taux d'anticorps anti-NO-cystéine au 6ème jour après l'induction et les valeurs de la force musculaire des membres postérieurs au cours de l'étude pour les rats EAE (n = a) du jour O au 26ème jour.

Chaque symbole ( ◆ ) représente la valeur r du coefficient de corrélation de Bravais Pearson. La ligne en pointillés représente le niveau significatif de 0,05 (df = 7).

Pour chaque valeur r, on représente le relevé dispersé des anti-NO-cystéine individuels contre la force musculaire des membres postérieurs en association avec la courbe de régression.

L'augmentation du coefficient de corrélation entre les anticorps anti-NO-cystéine au 6ème jour et la force musculaire durant l'EAE indique une dynamique fonction du temps.

De plus, la disparition de myéline et la perte de force musculaire des membres postérieurs au 24ème jour, sont corrélées de manière significative pour le 24ième jour (r = 0,72 ; p = 0,029) et le 26ème jour (r = 0,72 ; p = 0,027)

En conclusion, ces résultats montrent
1) la présence d'anticorps anti-NO-cystéine dans les échantillons de sérums de patients atteints de SEP ;
2) la présence de ces anticorps anti-NO-cystéine à un stade préclinique au cours d'une EAE chez le rat ; et que
3) le taux des anticorps anti-NO-cystéine est un marqueur précoce de l'évolution pathologique de la maladie.

### Exemple 3 : Etude clinique

Un groupe de 10 patients présentant une SEP évoluant par poussée, caractérisé par une activité clinique importante l'année précédent l'inclusion, a été suivi sur le plan clinique et biologique.

Les critères d'inclusions des 10 patients sont indiqués ci-après :

Il s'agit de patients
- présentant une SEP certaine selon les critères de Poser et al (5),
- ayant une forme rémittente avec ou sans séquelles selon Lublin et Reingold (6),
- ayant un handicap modéré mesuré selon l'échelle Expanded Disability Status Scale de Kurtzke (11) (EDSS), score EDSS < 5,5 à l'inclusion,
- présentant une SEP active, à savoir ayant eu au moins deux poussées dans les deux ans précédant l'inclusion,
- à plus d'un mois de la fin de la dernière poussée lors de l'inclusion,
- n'ayant pas pris de corticoïdes depuis au moins un mois au moment de l'inclusion,
- éventuellement sous un traitement de fond par azathioprine ou béta-interféron, mais ayant débuté depuis au moins 3 mois.
- ayant signé une feuille de consentement éclairé après avoir reçu une lettre les informant des objectifs de l'étude.

On procède tout d'abord à une évaluation clinique (vérification des critères d'inclusion, signature du consentement, examen neurologique pour mesure du score de l'EDSS et de l'index Ambulatoire de Hauser et al. (7) et Rine Hole peg test (9HPT) pour mesurer les fonctions motrices des membres supérieurs.

On effectue ensuite les prélèvements biologiques.

Un prélèvement de 0,5 ml de sang sur tube sec, par ponction digitale à l'inclusion, puis tous les 15 jours au domicile du patient par l'infirmière enquêtrice pendant toute la durée du suivi.

En cas de poussée clinique, on évalue le score EDSS maximum au cours de la poussée. Le traitement habituel des poussées a été effectué (méthylprednisolone 1 g/j/5j IV)

Un prélèvement sanguin a été effectué lors de cette évaluation clinique et le patient convoqué pour une évaluation clinique de contrôle un mois après la poussée (EDSS, Index ambulatoire, 9 HPT). Le suivi biologique est ensuite poursuivi jusqu'à la fin de l'étude.

### dosages

On a mesuré par ELISA le taux (DO) d'anticorps anti-NO cystéine selon la méthode ci-dessus.

Le criètre principal d'évaluation a consisté à rechercher une corrélation entre le dosage du taux d'anticorps anti-NO-cystéine (densité optique) d'une part et les 3 variables suivantes d'autre part :
- survenue d'une poussée dans les deux seamines suivantes ;
- en cas de poussée : EDSS maximum au cours de la poussée ;
- en cas de poussée : EDSS 1 mois après la poussée moins EDSS à l'inclusion.

Les variables établies à partir de l'index ambulatoire et du 9 HPT ont été utilisés comme critères accessoires d'évaluation.

Ces résultats démontrent l'intérêt de leur dosage lors du suivi des patients atteints de SEP afin de prédire la survenue et la sévérité des poussées. Leur application ouvre de nouvelles voies dans la recherche de traitement préventif de la SEP.

Dans une étude prospective, on effectuera alors avantageusement un suivi clinique et d'imagerie et le dosage de ces anticorps dans un groupe de patients atteints d'une SEP rémittente active.

### Exemple 4 : Identification d'antigènes protéiques nitrosylés de la myéline.

### a) Préparation de la protéine MOG recombinante.

On utilise 2 plasmides d'expression du type pGEX (Pharmacia) contenant respectivement la séquence codante complète de la MOG humaine ou la séquence codant pour la partie extracellulaire de la MOG (Dautigny, CNRS U1488, Paris).

Les sérums de patients correspondent à différentes catégories de SEP.

### 1. Préparation de bactéries compétentes

Des bactéries E. Coli de type XL1Blue sont rendues compétentes en fragilisant leur membrane grâce à du MgCl₂ 0,1 M, puis du CaCl₂ 50mM. Les bactéries sont alors extrêmement sensibles et il convient de procéder rapidement à la transformation.

### 2. Transformation de bactéries

Les bactéries sont mises en contact avec le plasmide pendant 30 min à 4°C. Pendant cette phase, l'ADN va se coller à la membrane bactérienne. Puis on procède à un choc thermique à 42°C pendant 1 min. L'ADN pénètre dans les bactéries. Celles-ci sont mises à incuber 1 h à 37°C pour régénérer, et enfin sont étalées sur un milieu sélectif LBAgar+Ampicilline. L'intégration du plasmide dans les bactéries leur confère la résistance à l'ampicilline.

### 3. Extraction plasmidique

Pour vérifier la présence du plasmide dans les colonies, une colonie isolée a été ensemencée dans 5 ml de LBAmp et mise à incuber la nuit à 37°C. Le lendemain, les bactéries lavées dans du TNE sont soumises à une lyse et une extraction au Phénol/Chloroforme/Alcool isoamylique dans un rapport volumique de 25/24/l. Après centrifugation, les protéines et l'ADN génomique se retrouvent à l'interface de la phase aqueuse et de la phase organique tandis que l'ADN plasmidique (comme l'ARN) est soluble dans la phase aqueuse. Cette phase est récupérée et les plasmides sont précipités avec des sels acétate d'ammonium (0,2 M final) et de l'éthanol absolu à -20°C. Une centrifugation à 12000 rpm permet de concentrer les plasmides dans le culot. Les plasmides sont ensuite lavés à l'éthanol 75°, mis à sécher, remis en suspension dans un faible volume d'eau, puis traités à la RNase.

Enfin, les plasmides sont digérés par EcoRI ou BamHI pour vérifier, après migration sur gel d'agarose ou de polyacrylamide, leur carte de restriction, et donc leur identité.

### 4. Induction de l'expression de pGEX

Une préculture de 5 ml de bactéries transformées avec l'un des deux plasmides est mise à incuber dans du LBAmp durant environ 14 h. On transfère ensuite progressivement cette pré-culture dans 20 puis 200 ml de LBAmp. Les bactéries sont de nouveau mises en culture à 37°C jusqu'à ce qu'elles atteignent leur phase exponentielle de croissance mesurée par une DO à 595 nm de 0,5. A ce stade, l'ajout d'isopropylthiogalactoside (IPTG), 5 mM final, est responsable de l'activation de la transcription de la séquence codante de la protéine de fusion. L'IPTG se fixe au promoteur pTac dérivé du gène de la β galactosidase et induit la transcription du cadre de lecture ouvert contenant l'insert : séquence de la MOG totale ou partielle et, à la suite, la séquence codante pour la glutathion S transférase, GST. La protéine résultante correspond à la fusion de la GST et de la MOG partielle ou totale. La culture est laissée 2h30 à incuber, le temps d'obtenir une quantité relativement importante de protéines.

### 5. Extraction de protéines

Les manipulations sont exécutées à 4°C.

Après centrifugation de la culture précédente (3000 rpm), le culot bactérien est repris dans un tampon de lyse 1 basique (1 ou 2 ml de tampon pour 50 ml de culture de départ). On ajoute 80 µl de lysosyme (10 mg/ml) et on laisse sur glace 20 min.

4 mg d'acide déoxycholique, sont ajoutés dans chaque préparation. Le mélange devient visqueux après agitation, l'ADN génomique est cassé par aspiration/refoulement avec une micropipette. 100 µl de DNase digèrent l'ADN, pendant 30 min. à température ambiante.

Le mélange ainsi obtenu correspond à des résidus de bactéries et à des protéines, solubilisées ou non, dans le tampon de lyse. Une première centrifugation à 12000 rpm, pendant au moins 1 heure, permet de séparer d'une part, les produits solubilisés dans le surnageant et d'autre part les débris, membranes et ADN résiduels, ainsi que les protéines non solubilisées dans le culot.

Le culot est remis en suspension dans un tampon de lyse 2 contenant un détergent : le Triton X100. Une deuxième centrifugation de 15 min. à 12000 rpm sépare les dernières protéines solubles du culot contenant des composants hydrophobes. Ce culot est remis en suspension dans 100 ou 200 µl d'eau et donne une dispersion.

### 6. Electrophorèse et Western-blot

L'électrophorèse en gel discontinu (gel de concentration, gel de séparation) permet de séparer les protéines suivant leur poids moléculaire uniquement, en présence de SDS.

A la suite de l'électrophorèse, on peut colorer avec une solution de bleu de Coomassie toutes les protéines qui ont migré sur le gel, ou on réalise un Western-blot qui, à la fin, permettra de ne révéler que certaines protéines.

Pour cette dernière technique, les protéines sont transférées du gel sur une membrane 〈Immobilon^{R}, Millipore) en appliquant un courant de 0,8 mA/cm² pendant 2 heures.

La membrane est lavée, saturée avec une solution contenant 3 % de lait écrémé. La membrane est mise à incuber, pendant environ 14 h, dans une solution contenant un premier anticorps reconnaissant le protéine de fusion.

Pour les contrôles, il s'agit du surnageant de culture d'hybridomes, fait à partir de lignées cellulaires de souris, et produisant des Ac monoclonaux anti GST.

La membrane est ensuite lavée, de nouveau saturée avec du lait 3 %, puis incubée 2 h avec un Ac secondaire qui reconnaît le premier Ac fixé sur la protéine. Cet Ac secondaire est un Ac anti Ig de souris couplé à la peroxydase.

Après un dernier lavage, le complexe protéine-Ac primaire-Ac secondaire est révélé par la réaction, en présence d'H₂O₂, entre la peroxydase et un chromogène le 4chloro-1-naphtol qui devient noir et précipite à l'endroit où la protéine a migré.

La méthodologie exposée ci-dessus permet de disposer de fragments de la MOG recombinante.

### b) Tests réalisés avec les protéines de fusion GST-MOG.

Afin de distinguer les différentes formes et stades évolutifs de SEP, on utilise des banques de sérums de patients contre ces protéines recombinantes nitrosylées.

Pour les tests de patients, le premier Ac correspond à une dilution au 100ème des sérums et le deuxième Ac est un Ac anti IgG, IgA, IgM humains, couplé à la peroxydase.

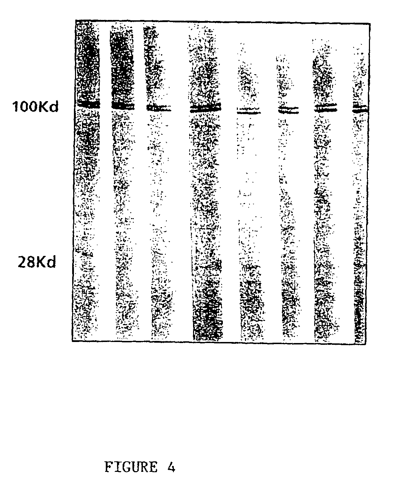

### c) A partir d'une préparation de myéline humaine qui a été nitrosylée in vitro, on a procédé à un criblage d'anticorps des différentes formes de SEP par Western blot. Deux protéines principales d'environ 100 kDa et de 25 kDa ont été révélées (figure 4).

Afin de caractériser ces protéines nitrosylées et rendues de ce fait immunogènes, des tests ont été réalisés sur des sérums de patients SEP contre la MAG nicrosylée, par ELISA/dot blot. Une première analyse de 58 sérums de patients atteints de SEP et de témoins montre une forte présence d'anticorps contre la MAG sous forme nitrosylée chez les patients de formes actives. Les patients en poussée (n=12) ou en progression évolutive (n=10) présentent un taux d'anticorps anti-MAG nitrosylée fortement élevé en comparaison avec les patients en situation stables (n=12), en rémission (n=12) et les sujets sains (n=12) (one way ANOVA ; F (4,53) = 22,261, p<0.0001).

### Références bibliographiques

1. A. I. Boullerne et al, J. Neuroimmunol. 60, 117-124 (1995)
2. A.I. Boullerne et al, J. Neuroimmunol. 65. 75-81 (1996)
3. J.S. Stamler et al, Proc. Natl. Acad. Sci. U.S.A. 89, 444 (1992)
4. H. Wekerle, Curr. Opin. Neurobiol. 3, 779 (1993)
5. C.M. Poser et al, Ann. Neurol. 13, 227-231 (1983)
6. F.D. Lublin et al, Neurology, 46, 907-911 (1996)
7. S.L. Hauser et al, N. Engl. J. Med., 308 ; 173-180 (1983)

## Revendications

1. Utilisation *in vitro* d'anticorps tels que développés durant une pathologie auto-immune inflammatoire du système nerveux central ou périphérique, ces anticorps appartenant au groupe constitué par les anticorps anti-NO-P, où P représente une protéine, une protéine sous forme recombinante, un peptide, ou un acide aminé, capable d'accepter NO, ou les anticorps anti-acides gras, comme marqueurs prédictifs d'une poussée clinique ou d'une nouvelle lésion de ladite pathologie.

2. Utilisation selon la revendication 1, **caractérisée en ce que**, dans les anticorps anti-NO-P, P représente une glycoprotéine associée à la myéline (MAG) ou une glycoprotéine majeure d'oligodendrocytes (MOG) ou, comme acide aminé, P représente un résidu cystéine.

3. Utilisation selon la revendication 1, **caractérisée en ce que**, dans les anticorps anti-acides gras, l'acide gras est choisi parmi l'acide laurique, l'acide palmitique, l'acide palmitoléique, l'acide stéarique, l'acide oléique, l'acide linoléique, l'acide arachidonique, l'acide eicosapentanoique et l'acide docosahexaénoïque.

4. Test de diagnostic précoce de la survenue de poussées et de lésions dans une pathologie auto-immune inflammatoire du système nerveux central ou périphérique, **caractérisé en ce qu'**il comprend la mise en contact d'un échantillon de sérum du patient avec un antigène nitrosylé NO-P, où P représente une protéine, une protéine sous forme recombinante, un peptide, ou un acide aminé capable d'accepter NO, ou la mise en contact d'un échantillon de sérum du patient avec un antigène représenté par un acide gras, et la révélation de la réaction antigène-anticorps lorsqu'elle s'est produite.

5. Test selon la revendication 4, **caractérisé en ce qu'**on incube le sérum et l'antigène à une température de 37°C.

6. Test selon la revendication 4 ou 5, **caractérisé en ce que** l'antigène est couplé à une protéine porteuse.

7. Test selon la revendication 6, **caractérisé en ce que** la protéine porteuse est KLH ou l'albumine de sérum bovin, utilisée sous forme délipidée lorsque le test est appliqué à la détermination d'anticorps anti-acide gras.

8. Test selon la revendication 6 ou 7, **caractérisé en ce que** la protéine porteuse est séparée de l'haptène par un bras d'écartement.

9. Test selon l'une quelconque des revendications 4 à 8 **caractérisé en ce que** l'antigène comprend comme protéine P un résidu cystéine et comme acide gras un acide choisi parmi l'acide laurique, l'acide palmitique, l'acide palmitoléique, l'acide stéarique, l'acide oléique, l'acide linoléique, l'acide arachidonique, l'acide eicosapentanoïque et l'acide docosahexaénoique.

10. Test selon l'une ' quelconque des revendications 4 à 9, **caractérisé en ce qu'**on révèle les anticorps fixés selon la méthode ELISA, lesdits antigènes étant fixés à un support solide et un deuxième anticorps étant ajouté au milieu biologique.

## Patentansprüche

1. Verwendung *in vitro* von Antikörpern, wie sie während einer entzündlichen Auto-Immun-Pathologie des Zentralnervensystems oder peripheren Nervensystems gebildet werden, wobei diese Antikörper zu der Gruppe gehören, die aus den Anti-NO-P-Antikörpern besteht, worin P ein Protein, ein Protein in rekombinanter Form, ein Peptid oder eine Aminosäure, die NO akzeptieren kann, oder den Anit-Fettsäuren-Antikörpern besteht, als Frühindikatoren, welche einen klinischen Schub oder eine neue pathologische Schädigung ankündigen.

2. Verwendung nach Anspruch 1, **dadurch gekennzeichnet, daß** in den Anti-NO-P-Antikörpern P ein mit Myelin assoziiertes Glykoprotein (MAG) oder ein Hauptglykoprotein der Oligodendrocyten (MOG) oder als Aminosäure P einen Cysteinrest bedeutet.

3. Verwendung nach Anspruch 1, **dadurch gekennzeichnet, daß** in den Anti-Fettsäuren-Antikörpern die Fettsäure ausgewählt ist unter Laurinsäure, Palmitinsäure, Palmitoleinsäure, Stearinsäure, Oleinsäure, Linolensäuse, Arachidonsäure, Eicosapentansäure und Docosahexaensäure.

4. Frühdiagnosetest des Auftretens von Schüben und Schädigungen in einer entzündlichen Auto-Immun-Pathologie des Zentralnervensystems oder peripheren Nervensystems, **dadurch gekennzeichnet, daß** bei ihm eine Serumprobe des Patienten in Berührung gebracht wird mit einem nitrosylierten Antigen NO-P, worin P ein Protein, ein Protein in rekombinanter Form, ein Peptid oder eine Aminosäure, die NO akzeptieren kann, bedeutet, oder eine Serumprobe des Patienten mit einem durch eine Fettsäure repräsentierten Antigen in Berührung gebracht wird und die Antigen-Antikörperreaktion festgestellt wird, wenn sie eintritt.

5. Test nach Anspruch 4, **dadurch gekennzeichnet, daß** man das Serum und das Antigen bei einer Temperatur von 37°C inkubiert.

6. Test nach Anspruch 4 oder 5, **dadurch gekennzeichnet, daß** das Antigen an ein Trägerprotein gekoppelt ist.

7. Test nach Anspruch 6, **dadurch gekennzeichnet, daß** das Trägerprotein KLH oder Rinderserum-Albumin ist, das in entfetteter Form verwendet wird, wenn der Test zur Bestimmung von Anti-Fettsäure-Antikörpern angewandt wird.

8. Test nach Anspruch 6 oder 7, **dadurch gekennzeichnet, daß** das Trägerprotein durch einen Spacer vom Hapten getrennt ist.

9. Test nach einen der Ansprüche 4 bis 9, **dadurch gekennzeichnet, daß** das Antigen als Protein P einen Cysteinrest und als Fettsäure eine Säure umfaßt, die ausgewählt ist unter Laurinsäure, Palmitinsäure, Palmitoleinsäure, Stearinsäure, Oleinsäure, Linolensäure, Arachidonsäure, Eicosapentansäure und Docosahexaensäure.

10. Test nach einem der Ansprüche 4 bis 9, **dadurch gekennzeichnet, daß** man die fixierten Antikörper nach der ELISA-Methode ermittelt, wobei die Antigene an einem festen Träger fixiert sind und ein zweiter Antikörper dem biologischem Milieu zugesetzt wird.

## Claims

1. *In vitro* use of antibodies as developed in inflammatory auto-immune pathology of the central or peripheral nervous system, these antibodies belonging to the group consisting of anti-NO-P antibodies wherein P represents a protein, a protein in recombinant form, a peptide or an amino-acid capable of accepting NO or anti-fatty acid antibodies, as predictive markers of a clinical outbreak or a new lesion of said pathology.

2. Use according to claim 1, **characterised in that**, in anti-NO-P antibodies, P represents a glycoprotein associated with myelin (MAG) or a major glycoprotein of oligodendrocytes (MOG) or, as an amino-acid, P represents a cysteine residue.

3. Use according to claim 1, **characterised in that**, in anti-fatty acid antibodies, the fatty acid is selected from lauric acid, palmitic acid, palmitaleic acid, stearic acid, oleic acid, linoleic acid, arachidonic acid, eicosapentanoic acid and docosahexaenoic acid.

4. Early diagnostic test on the occurrence of outbreaks and lesions in inflammatory auto-immune pathology of the central or peripheral nervous system, **characterised in that** it involves bringing a sample of the patient serum into contact with a nitrosylated antigen NO-P, wherein P represents a protein, a protein in recombinant form, a peptide or an amino-acid capable of accepting NO or bringing a sample of the patient serum into contact with an antigen represented by a fatty acid, and the revelation of the antigen/antibody reaction when it has occurred.

5. Test according to claim 4, **characterised in that** the serum and the antigen are incubated at a temperature of 37°C.

6. Test according to claim 4 or 5, **characterised in that** the antigen is coupled to a carrier protein.

7. Test according to claim 6, **characterised in that** the carrier protein is KLH or bovine serum albumin, used in delipidated form when the test is applied to the determination of anti-fatty acid antibodies.

8. Test according to claim 6 or 7, **characterised in that** the carrier protein is separated from the hapten by a spacer arm.

9. Test according to any one of claims 4 to 8, **characterised in that** the antigen comprises a cysteine residue as the protein P and an acid selected from lauric acid, palmitic acid, palmitoleic acid, stearic acid, oleic acid, linoleic acid, arachidonic acid, eicosapentanoic acid and docosahexaenoic acid as the fatty acid.

10. Test according to any one of claims 4 to 9, **characterised in that** the antibodies fixed by the ELISA method are revealed, said antigens being fixed to a solid support and a second antibody being added to the biological environment.
